# EUROPEAN PATENT SPECIFICATION

(11) **EP 0 843 998 B1**
(45) Date of publication and mention of the grant of the patent: **13.02.2002**
(21) Application number: 97120067.0
(22) Date of filing: 17.11.1997
(51) Int. Cl.: A61K 9/10, A61K 31/54, A61K 31/63

(54) **Oral pharmaceutical composition having antipyretic, analgesic and antiinflammatory activity**
Orale pharmazeutische Zubereitung mit antipyretischer, analgetischer und entzündungshemmender Wirkung.
Composition pharmacuetique orale ayant une activité antipyrétique, analgésique et antiinflammatoire.

(30) Priority: 20.11.1996 IT MI962423
(43) Date of publication of application: 27.05.1998
(73) Proprietor: Leetrim Ltd., Dublin, London WC2A 3LJ (GB); Sintactica Srl, 20060 Cassina de'Pecchi (MI) (IT)
(72) Inventor: Borsa, Massimiliano, 20090 Vimodrone (MI) (IT)
(74) Representative: Beneduce, Gianna

(56) References cited:
- EP-A- 0 196 434
- EP-A- 0 441 307
- GB-A- 2 172 487
- US-A- 4 824 841

## Description

The object of the present invention consists of a pharmaceutical composition, suitable for oral administration, which finds use as antipyretic, analgesic and antiinflammatory drug.

More particularly said pharmaceutical composition for oral administration contains, as active principle, in a suitable mixture of excipients, a nonsteroidal antiinflammatory compound, preferably a compound, containing a sulphonamide group, selected from the group consisting of N-(4-nitro-2-phenoxyphenyl)methanesulphonamide or Nimesulide, 4-hydroxy-2-methyl-N-2-pyridinyl-2H-1,2-benzothiazine-3-carboxamide-1,1-dioxide or Piroxicam and 4-hydroxy-2-methyl-N-2-pyridinyl-2H-thieno[2,3-e]-1,2-thiazine-3-carboxamide-1,1-dioxido or Tenoxicam.

The nonsteroidal antiinflammatory compounds or FANS have antipyretic, analgesic and antiinflammatory activity and are generally used in oral and parenteral administration in the form of tablets, capsules, suppositories or vials. Their action develops by inhibiting the synthesis of the prostagladins at the cycloxygenase level in different districts, therefore they may show a different ratio between antipyretic activity, analgesic activity and antiinflammatory activity. The clinical data demonstrate their activity in reducing the symptoms in pathological conditions characterized by phlogosis, hyperpyrexia and pain.

The pain threshold of the patient, the entity of the hyperthermia or the inflammatory conditions to be treated as well as the general conditions of the patient (age, sex, weight, psychological conditions, ecc.) suggest the opportunity to have at disposal a pharmaceutical form by which it is possible to modulate the activity of the antiinflammatory product so that only the minimum effective dose, among the accepted dosages, be administrated to the patient to cure his condition.

It is therefore desirable to have at disposal pharmaceutical compositions containing the antiinflammatory product in such a phase that, as the liquid phase, allows to suitably fractionate the full dose so that to administrate, each time, the effective exact amount of the active principle.

It is known that the nonsteroidal antiinflammatory compounds are not usually used in liquid pharmaceutical forms suitable to be administered by oral route, because they, when dissolved in suitable solvents give solutions which are characterized by a very bad taste, certainly not suitable for the oral administration, while the same, when suspended in suitable media give suspension which show an unsatisfactory stability.

The object of the present invention is to provide a liquid pharmaceutical composition suitable for the oral administration in which the active principle, consisting of a nonsteroidal antiinflammatory compound, is contained, in suspension in a suitable medium, and that said suspension has a good taste and a satisfactory stability.

Said liquid pharmaceutical composition suitable for the oral administration is represented by the composition which is described hereafter in the Examples, said composition containing, as active principle, a nonsteroidal antiinflammatory compound, in a suitable form, in a suitable mixture of excipients such as humectant/surfactant agents, suspending agents, sweetening agents, opacifying agents, preservative agents, optionally buffer agents and aromatizing agents and it is characterized by the fact that the nonsteroidal antiinflammatory compound, in a micronized form having a granulometry preferably lower than 5µ for an amount over 95% in weight, it is therein included in a quantity from 1 to 5% in weight in respect to the volume of the suspension, by having a pH between 3.5 and 6, by having a low density, generally between 1.05 and 1.20 and that the suspending agent is represented by mixtures suitably equilibrated of suspending agents.

As humectant/surfactant agents, non ionic surfactants such as polysorbate 20, polysorbate 80 (International Non-proprietary Name for sorbitan mono-oleate polyoxyethylene), Span (Trademark of ICI for sorbitan esters) in the concentration range 0.1 - 0.5% or ionic surfactants such as sodium laurylsulfate in the range 0.1 - 0.3% can be used.

As suspending agents, a mixture of suspending agents such as cellulose, for example hydroxypropylethylcellulose, hydroxypropylmethylcellulosa, sodium carboxymethylcellulose or other polysaccharides, for example xanthan gum, pectins, carrageenin, Avicel RC 591 (Trademark of FMC Corporation for crystalline cellulose) may be used. Preferably ternary mixtures of suspending agents are used, more preferably the mixture consisting of hydroxypropylmethylcellulose/Avicel RC 591 (Trademark of FMC Corporation for crystalline cellulose)/xanthan in the ratio from 1:4:1 to 1:10:2.5.

As buffer agent, citrate buffers at pH between 3 and 5 having molarity between 0.1 and 0.5 and phosphate buffers at pH between 5 and 6 can be used.

As sweetening agent, 30-50% of sucrose can be used, which gives to the formulation, besides a good taste, also a density which improves the stability of the suspension. This effect can also be obtained with 30-40% of sorbitol or gylcerol or in combination with sucrose.

As aromas the orange, tangerine, grapefruit aromas, cherry or wild fruit aromas are indicated. The suitable quantities are from 0.05 to 0.2 % according to the concentration of use of the aromas themselves and to their concentration in the aromatic substances.

As opacifying agent, titanium dioxide can be used which makes the suspension opaque.

As preservative agent, methyl and propyl parahydroxybenzoates and/or sorbate can be used. Quantities from 10 to 30 mg/100 ml of parahydroxybenzoates, for a ratio from 3:1 to 9:1 of methyl:propyl, are recommended, the appropriate quantities of sorbates are from 50 and 100 mg per 100 ml.

The Examples which follow serve to describe the invention in a clearer manner.

### Example 1

Composition of 100 ml of pharmaceutical composition in the form of a suspension.

| | |
|---|---|
| Nimesulide | 1.000 g |
| Sucrose | 20.000 g |
| Sorbitol | 15.000 g |
| Methylparaben | 0.100 g |
| Propylparaben | 0.020 g |
| Xanthan gum | 0.400 g |
| Sodium laurylsulfate | 0.010 g |
| Orange aroma | 0.200 g |
| Titanium dioxide | 0.100 g |
| Citrate buffer 0.1M, pH5 | 20.000 ml |
| Distilled water to | 100.000 ml |

In about 50% of the final volume of the distilled water at 80-90°C are added, under stirring, methylparaben and propylparaben and, after having cooled the mixture to 50-60°C, xanthan gum and sucrose. The mixture is left to swell keeping it under stirring and sorbitol, sodium laurylsulfate and then the micronized nimesulide (96% by weight, size 4µ) are added and, afterwards, in sequence, citrate buffer, titanium dioxide and the aroma.

It is brought to the final volume with distilled water and the mixture is homogenised in a turboemulsifier for a period of 5-10 minutes, then the suspension obtained is left to disareated and divided into separate packages.

### Example 2

Components of 100 ml of pharmaceutical composition in the form of a suspension.

| | |
|---|---|
| Nimesulide | 5.00 g |
| Polysorbate 20 (International Non-proprietary Name for sorbitan mono-oleate polyoxyethylene) | 0.10 g |
| Hydroxypropylmethyl cellulose | 0.10 g |
| Avicel RC591 (Trademark of FMC Corporation for crystalline cellulose) | 0.80 g |
| Xanthan gum | 0.15 g |
| Sucrose | 30.00 g |
| Glycerol | 5.00 g |
| Methylparaben | 0.12 g |
| Propylparaben | 0.03 g |
| Acerole aroma | 0.40 g |
| Titanium dioxide | 0.50 g |
| Distilled water to | 100 ml |

In about 50% of the final volume of the distilled water, at 80-90°C, methylparaben and propylparaben and sucrose are added under stirring. After having cooled the mixture to 50-60°C, hydroxypropylmethylcellulose, Avicel RC591 (Trademark of FMC Corporation for crystalline cellulose) and xanthan gum are added. The mixture is left to swell keeping it under stirring, then glycerol and polysorbate 20 (International Non-proprietary Name for sorbitan mono-oleate polyoxyethylene) and the micronized nimesulide (96%, size 4.5µ) and afterwards, in sequence, titanium dioxide and the aroma are added thereto. Operation is carried out as previously indicated in Example 1 till the preparation of the final packages.

### Example 3

Components of 100 ml of pharmaceutical composition in the form of a suspension.

| | |
|---|---|
| Piroxicam | 1.000 g |
| Sucrose | 20.000 g |
| Sorbitol | 15.000 g |
| Methylparaben | 0.100 g |
| Propylparaben | 0.020 g |
| Xanthan gum | 0.300 g |
| Sodium laurylsulfate | 0.010 g |
| All fruit aroma | 0.200 g |
| Titanium dioxide | 0.100 g |
| Citrate buffer pH 5 | 10 ml |
| Distilled water to | 100 ml |

Operation is carried out as indicated for the preparation of the compound of Example 1 with the difference that the active principle is represented by micronized Piroxicam.

### Example 4

Components of 100 ml of pharmaceutical composition in the form of a suspension.

| | |
|---|---|
| Piroxicam | 2.00 g |
| Methylparaben | 0.12 g |
| Propylparaben | 0.04 g |
| Sorbitol 70% | 40.00 g |
| Hydroxypropylmethylcellulose | 0.25 g |
| Sodium carboxymethyl cellulose | 0.20 g |
| Span 60 (Trademark of ICI for sorbitan esters) | 0.150 g |
| Acerola aroma | 0.250 g |
| Titanium dioxide | 0.150 g |
| Distilled water to | 100 ml |

Operation is carried out as previously described in Example 1, by first mixing methylparaben and propylparaben then adding hydroxypropylmethyl cellulose and sodium carboxymethyl cellulose and afterwards sorbitol and Span 60 (Trademark of ICI for sorbitan esters). The active principle, consisting of piroxicam, is then added followed by titanium dioxide and the aroma, and it is completed as previously described.

## Claims

1. An oral pharmaceutical composition in the form of a suspension having antipyretic, analgesic and antiinflammatory activity, **characterized by** the fact that it contains as an active principle a nonsteroidal antiinflammatory compound, in a micronized form, over 95% in weight of granulometry lower than 5µ, in a concentration from 1 to 5% by weight of the total volume of the suspension, a suitable humectant surfactant agent, a suitable mixture of suspending agents, a suitable sweetening agent, a suitable opacifying agent, a suitable preservative agent, a suitable aromatizing agent, optionally a suitable buffer agent and that it presents a density between 1.05 and 1.20 and a pH between 3 and 6.

2. An oral pharmaceutical composition in the form of a suspension according to claim 1, **characterized by** the fact that the antiinflammatory compound is selected from the group consisting of Nimesulide, Piroxicam and Tenoxicam.

3. An oral pharmaceutical composition in the form of a suspension according to claim 1 or 2, **characterized by** the fact that the mixture of the suspending agents is a suitable mixture of agents selected form the group consisting of hydroxypropylethylcellulose, hydroxypropylmethylcellulose, sodium carboxymethylcellulose, xanthan gum, pectin, carrageenin, Avicel RC 591 (Trademark of FMC Corporation for crystalline cellulose), that the humectant/surfactant agent is a suitable ionic or non-ionic surfactant in a concentration 0.1-0.5% by weight per volume of the suspension, that the sweetening agent is selected from the group consisting of sucrose, sorbitol and glycerol or consisting of their mixture in a concentration 30-50% by weight per volume of the suspension and that the aromitizing agents are selected from the orange, tangerine, grapefruit, cherry or wild fruit aromas in concentrations from 0.05 to 0.2% by weight per volume of the emulsion.

4. An oral pharmaceutical composition in the form of a suspension according to claim 3, **characterized by** the fact that the antiinflammatory compound is Nimesulide and that the mixture of suspending agents is a ternary mixture consisting in hydroxypropylmethylcellulose: Avicel RC 591 (Trademark of FMC Corporation for crystalline cellulose): xanthan in the ratio from 1:4:1 to 1:10:2.5 and that the sweetening agent is formed by a glycerol:sucrose mixture from 1:4 - 1:7.

## Patentansprüche

1. Eine orale pharmazeutische Zusammensetzung in Form einer Suspension mit fiebersenkender, schmerzlindernder und entzündungshemmender Aktivität, **gekennzeichnet durch** die Tatsache, dass sie als einen aktiven Grundstoff eine nichtsteroide entzündungshemmende Verbindung in mikronisierter Form, wobei mehr als 95 Gew.-% in der Teilchengrößenanalyse größer als 5 µ sind, in einer Konzentration von 1 bis 5 Gew.-% des Gesamtvolumens der Suspension, ein geeignetes oberflächenaktives Befeuchtungsmittel, eine geeignete Mischung von Suspendierhilfen, ein geeignetes Süßungsmittel, ein geeignetes Trübungsmittel, ein geeignetes Konservierungsmittel, einen geeigneten Aromastoff, fakultativ einen geeigneten Puffer enthält, und **dadurch**, dass sie eine Dichte zwischen 1,05 und 1,20 und einen pH-Wert zwischen 3 und 6 aufweist.

2. Eine orale pharmazeutische Zusammensetzung in Form einer Suspension gemäss Anspruch 1, **gekennzeichnet durch** die Tatsache, dass die entzündungshemmende Verbindung ausgewählt ist aus der Gruppe bestehend aus Nimesulid, Piroxicam und Tenoxicam.

3. Eine orale pharmazeutische Zusammensetzung in Form einer Suspension gemäss Anspruch 1 oder 2, **gekennzeichnet durch** die Tatsache, dass die Mischung der Suspendiermittel eine geeignete Mischung von Agenzien ist, die ausgewählt sind aus der Gruppe, die aus Hydroxypropylethylcellulose, Hydroxypropylmethylcellulose, Natriumcarboxymethylcellulose, Xanthangummi, Pektin, Carrachenin, Avicel RC 591 (Marke der FMC Corporation für kristalline Cellulose) besteht, dass das Befeuchtungs-/oberflächenaktive Mittel ein geeignetes ionisches oder nichtionisches oberflächenaktives Mittel in einer Konzentration von 0,1 bis 0,5 Gew.-% pro Volumen der Suspension ist, dass das Süßungsmittel ausgewählt ist aus der Gruppe bestehend aus Sucrose, Sorbitol und Glycerin, oder aus deren Mischung besteht, in einer Konzentration von 30 bis 50 Gew.-% pro Volumen der Suspension und dass die Aromastoffe ausgewählt sind aus Orangen-, Tangerin-, Grapefruit-, Kirsch- oder Wildfruchtaromen in Konzentrationen von 0,05 bis 0,2 Gew.-% pro Volumen der Suspension.

4. Eine orale pharmazeutische Zusammensetzung in Form einer Suspension gemäss Anspruch 3, **gekennzeichnet durch** die Tatsache, dass die entzündungshemmende Verbindung Nimesulid ist und dass die Mischung der Suspendiermittel eine ternäre Mischung ist, die aus Hydroxypropylmethylcellulose:Avicel RC 591 (Marke der FMC Corporation für kristalline Cellulose):Xanthan im Verhältnis von 1:4:1 bis 1:10:2,5 besteht, und dass das Süßungsmittel **durch** eine Mischung aus Glycerin:Sucrose im Verhältnis von 1:4 bis 1:7 gebildet wird.

## Revendications

1. Composition pharmaceutique orale, sous la forme d'une suspension ayant une activité antipyrétique, analgésique et anti-inflammatoire, **caractérisée en ce qu'**elle contient comme principe actif un composé anti-inflammatoire non stéroïde, sous forme micronisée, dont plus de 95% en poids de la granulométrie a une taille inférieure à 5 µm, en concentration de 1% à 5% en poids du volume total de la suspension, un agent tensioactif humectant adéquat, un mélange adéquat d'agents de mise en suspension, un agent édulcorant adéquat, un agent opacifiant adéquat, un agent conservateur adéquat, un agent aromatisant adéquat, facultativement un agent tampon adéquat, et **en ce qu'**elle présente une densité entre 1,05 et 1,20 et un pH entre 3 et 6.

2. Composition pharmaceutique orale sous la forme d'une suspension selon la revendication 1, **caractérisée en ce que** le composé anti-inflammatoire est choisi dans le groupe composé du Nimesulide, du Piroxicam et du Tenoxicam.

3. Composition pharmaceutique orale sous la forme d'une suspension selon la revendication 1 ou 2, **caractérisée en ce que** le mélange des agents de mise en suspension est un mélange adéquat d'agents choisis dans le groupe constitué de l'hydroxypropyléthylcellulose, de l'hydroxypropylméthylcellulose, de la carboxyméthyl-cellulose sodique, de la gomme de xanthane, de la pectine, de la carrageenine, de l'Avicel RC 591 (marque commerciale de la FMC Corporation pour la cellulose cristalline), **en ce que** l'agent tensioactif/humectant est un agent tensioactif ionique ou non ionique en concentration de 0,1-0,5% en poids par volume de la suspension, **en ce que** l'agent édulcorant est choisi dans le groupe constitué de la saccharose, du sorbitol et du glycérol ou constitué de leur mélange en concentration de 30-50% en poids par volume de la suspension, et **en ce que** les agents aromatisants sont choisis parmi les arômes d'orange, de mandarine, de pamplemousse, de cerise ou de fruits sauvages en concentrations de 0,05% à 0,2% en poids par volume de l'émulsion.

4. Composition pharmaceutique orale sous la forme d'une suspension selon la revendication 3, **caractérisée en ce que** le composé anti-inflammatoire est le Nimesulide, **en ce que** le mélange d'agents de mise en suspension est un mélange ternaire constitué d'hydroxypropylméthylcellulose:Avicel RC 591 (marque du commerce de la FMC Corporation pour la cellulose cristalline) :xanthane dans le rapport de 1:4:1 à 1:10:2,5 et **en ce que** l'agent édulcorant est formé d'un mélange de glycérol et saccharose de 1:4 à 1:7.
